# EUROPEAN PATENT SPECIFICATION

(11) **EP 1 812 092 B1**
(45) Date of publication and mention of the grant of the patent: **21.10.2015**
(21) Application number: 05778775.6
(22) Date of filing: 12.05.2005
(51) Int. Cl.: A61L 31/18

(54) **MEDICAL DEVICES COMPRISING POLYOXOMETALATES**
POLYOXOMETALATE ENTHALTENDE MEDIZINPRODUKTE
DISPOSITIFS MEDICAUX COMPRENANT DES POLYOXOMETALATES

(30) Priority: 10.11.2004 US 985242
(43) Date of publication of application: 01.08.2007
(73) Proprietor: Boston Scientific Limited, Hamilton HM11 (BM)
(72) Inventor: ATANASOSKA, Ljiljana, Edina, MN 55436 (US); WEBER, Jan, Maple Grove, MN 55311 (US); MILLER, Matthew, J., White Bear Lake, MN 55110 (US)
(74) Representative: Peterreins Schley
(86) International application number: PCT/US2005/016599
(87) International publication number: WO 2006/052284

(56) References cited:
- DE-U1- 20 112 762
- RHULE J T ET AL: "POLYOXOMETALATES IN MEDICINE" CHEMICAL REVIEWS, ACS,WASHINGTON, DC, US, vol. 98, no. 1, 1998, pages 327-357, XP000730500 ISSN: 0009-2665

## Description

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, stent-grafts, and covered stents.

An endoprosthesis can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

When the endoprosthesis is advanced through the body, its progress can be monitored, e.g., tracked, so that the endoprosthesis can be delivered properly to a target site. After the endoprosthesis is delivered to the target site, the endoprosthesis can be monitored to determine whether it has been placed properly and/or is functioning properly.

Methods of tracking and monitoring a medical device include X-ray fluoroscopy and magnetic resonance imaging (MRI). MRI is a non-invasive technique that uses a magnetic field and radio waves to image the body. In some MRI procedures, the patient is exposed to a magnetic field, which interacts with certain atoms, e.g., hydrogen atoms, in the patient's body. Incident radio waves are then directed at the patient. The incident radio waves interact with atoms in the patient's body, and produce characteristic return radio waves. The return radio waves are detected by a scanner and processed by a computer to generate an image of the body.

DE 201 12 762 U1 relates to endoprostheses comprising a gold-copper based alloy to increase its visibility in magnetic resonance imaging

### SUMMARY

The invention features medical devices in the form of endoprostheses having good MRI compatibility and methods of making the devices. The endoprostheses comprise a plurality of polyoxometalates, as set forth in the claims. Further aspects of the invention are described in the features of the dependent claims.

In some embodiments, the medical device includes a multi-layered structure having a plurality of first layers including molecular magnetic clusters, and a plurality of charged second layers. The magnetic clusters include polyoxometalates, and the charged second layers can include polyelectrolytes. The multi-layered structure is capable of magnetically shielding the medical device, thereby allowing the MRI visibility within the medical device to be enhanced.

Embodiments may include one or more of the following features. The device has a layer including the polyoxometalates. The device has a plurality of layers including the polyoxometalates, the layers being spaced from each other. The device further includes a first layer having a polyelectrolyte, the first layer being between the layers having the polyoxometalates. The device has a layer having the polyoxometalates electrostatically bonded to a layer having one or more charges. The device has a first layer having a first polymer, a second layer having a second polymer, and a third layer having the polyoxometalates between the first layer and the second layer, wherein the first polymer and the second polymer are covalently bonded to each other.

The polyoxometalates can be ferromagnetic, superparamagnetic, paramagnetic, or diamagnetic. The polyoxometalates can include an element selected from the group consisting of cobalt, iron, manganese, chromium, nickel, ruthenium, copper, and bismuth. The polyoxometalates can have at least one dimension of about 0.5 nm to about 50 nm. The polyoxometalates can be surrounded by a polymer. The polymer can be selected from the group consisting of polypyrrole, polythiophene, and polyaniline.

In another aspect, the invention features an endoprosthesis including a first layer having a plurality of polyoxometallates, and a second layer comprising a polyelectrolyte on the first layer.

Embodiments may include one or more of the following features. The endoprosthesis has a plurality of first layers and a plurality of second layers. At least two of the first layers are spaced from each other by one or more second layers. The polyoxometalates are ferromagnetic, paramagnetic, diamagnetic, or superparamagnetic. The polyoxometalates have an element selected from the group consisting of cobalt, iron, manganese, chromium, nickel, ruthenium, copper, and bismuth. The polyoxometalates have at least one dimension of about 0.5 nm to about 50 nm.

In another aspect, the invention features an endoprosthesis comprising polyoxometalates surrounded by a polymer. The polymer can be selected from the group consisting of polypyrrole, polythiophene, and polyaniline.

In another aspect, the invention features a method of making an endoprosthesis, the method including forming on the device a first layer having one or more positive charges, and forming on the first layer a second layer having polyoxometalates.

Embodiments may include one or more of the following features. The method further includes forming a plurality of first layers and a plurality of second layers. The first layer includes a polyelectrolyte. The polyoxometalates are ferromagnetic, diamagnetic, paramagnetic or superparamagnetic. The polyoxometalates includes an element selected from the group consisting of cobalt, iron, manganese, chromium, nickel, ruthenium, copper, and bismuth.

In another aspect, the invention features a method of making an endoprosthesis, the method comprising forming a composition on an endoprosthesis, the composition comprising a polymer surrounding a plurality of polyoxometalates.

Embodiments may include one or more of the following features. The method includes electropolymerizing a mixture having monomers to form the composition. The polymer is selected from the group consisting of polypyrrole, polythiophene, and polyaniline. The polyoxometalates are ferromagnetic, diamagnetic, paramagnetic, or superparamagnetic. The polyoxometalates include an element selected from the group consisting of cobalt, iron, manganese, chromium, nickel, ruthenium, copper, and bismuth. The polyoxometalates has at least one dimension of about 0.5 mm to about 50 mm.

Embodiments may include one or more of the following advantages. For example, while it is possible to obtain an image of material in the lumen of the stent by increasing the incident radiofrequency energy, this energy can pose a risk to the body (e.g., by heating the body). By magnetically shielding the stent as described herein, the incident radiofrequency energy can be reduced, thereby reducing the risk to the body.

The magnetically shielding structure can be flexible, which reduces cracking and facilitate adaptation to a medical device. The fabrication of the magnetically shielding structure can be well controlled to tailor, for example, the composition of the structure and the physical parameters of the structure. The structure can include a therapeutic agent, a radiopaque material, and/or a reinforcement aid. In some embodiments, the polyoxometalates are capable of transitioning to multiple oxidation states, which can provide catalytic properties. The structure can be applied to a variety of medical devices.

Other aspects, features and advantages will be apparent from the description of the preferred embodiments and from the claims.

### DESCRIPTION OF DRAWINGS

Fig. 1 is a perspective view of an embodiment of an endoprosthesis.
Fig. 2 is a detailed cross-sectional illustration of the endoprosthesis of Fig. 1, taken along line 2-2.
Fig. 3 is a flow chart of an embodiment of a method of making a stent.

### DETAILED DESCRIPTION

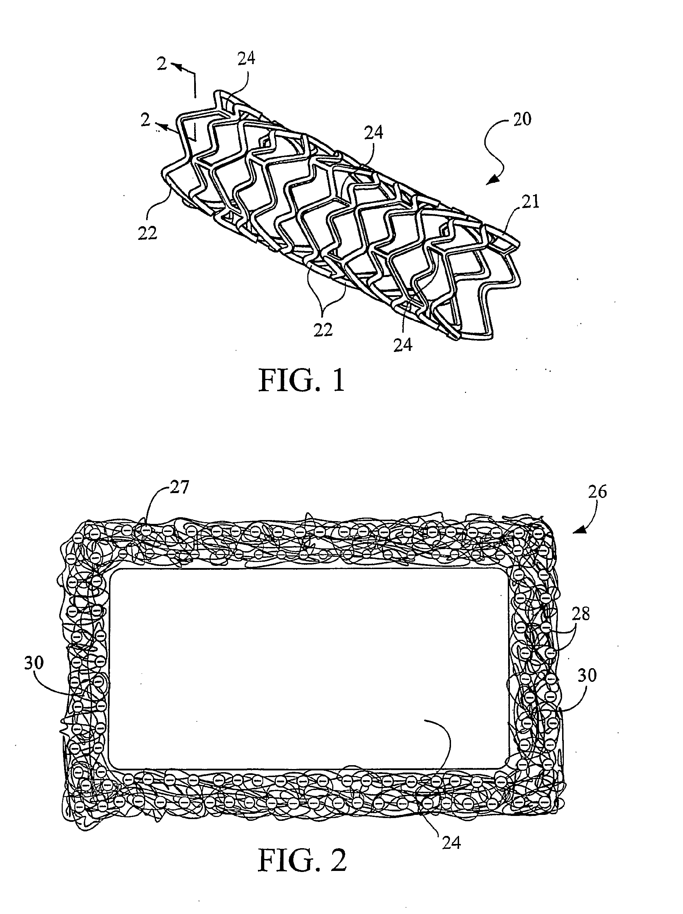

Referring to Fig. 1, a stent 20 having enhanced compatibility with magnetic resonance imaging (MRI) is shown. Stent 20 has the form of a tubular structure 21 defined by a plurality of bands 22 and a plurality of connectors 24 that extend between and connect adjacent bands. Referring to Fig. 2, stent 20 (as shown, a portion of connector 24) is coated with a multi-layered structure 26 that includes a plurality of layers 27 containing discrete and spaced magnetic clusters 28 (e.g., polyoxometalates) and a plurality of charged layers 30 (e.g., containing polyelectrolytes). Multi-layered structure 26, in particular layers 27 containing magnetic clusters 28, is capable of magnetically shielding stent 20 from high frequency electromagnetic fields (e.g., on the order of megahertzs) applied during MRI procedures, and enhancing the MRI visibility of material in the lumen of the stent, such as flowing blood or a blood clot.

During MRI, the induced currents in the stent can interfere with the incident electromagnetic field to reduce (e.g., to eliminate) the visibility of material in the lumen of the stent. More specifically, the magnetic environment of the stent can be constant or variable, such as when the stent moves within the magnetic field (e.g., from a beating heart) or when the incident magnetic field is varied. When there is a change in the magnetic environment of the stent, which can act as a coil or a solenoid, an induced electromotive force (emf) is generated, according to Faraday's Law. The induced emf in turn can produce an eddy current that induces a magnetic field that opposes the change in magnetic field. The induced magnetic field can reduce or enhance the incident magnetic field. The visibility of material in the lumen of the stent during MRI can depend, among other things, on the number of atomic spin transitions per unit volume, which can be directly related to the energy of the incident magnetic field. A similar (but much stronger) effect can be caused by a radiofrequency pulse applied during MRI.

By forming stent 20 to include layers 27 of magnetic clusters 28, the occurrence of an eddy current can be reduced (e.g., eliminated). More specifically, during MRI, soft magnetic clusters 28 are capable of redirecting part of the incident magnetic flux around the conductive tubular structure of the stent. This redirection can reduce the eddy currents from being generated linearly with the change in flux. Both the redirected magnetic flux (now capable of passing around the stent struts and into the interior of the stent) and the reduction in opposing magnetic field by the reduction of the eddy current can cause more spins in the interior of the stent to be excited. As a result, the MRI visibility of the material in the lumen of the stent can be enhanced.

As indicated above, magnetic clusters 28 include polyoxometalates. Polyoxometalates are nanosized inorganic oxygen clusters, such as inorganic metal oxygen clusters, that can be strictly uniform at the atomic level. The clusters are discrete molecular units with well-defined molecular formulas, in contrast to nanosized particles having infinite, extended lattice structures. Depending on the number of different types of non-oxygen atoms, polyoxometalates can be classified as isopolyanions or heteropolyanions. Isopolyanions can be described by the formula [MₓO_{y}]^{-p}; and heteropolyanions can be described by the formula [XₐM_{b}O_{c}]^{-q}, where M and X are different metals. Examples of metals include molybdenum, tungsten, vanadium, niobium, tantalum, cobalt, iron, ruthenium, titanium, nickel, chromium, platinum, zirconium, iridium, silicon, and boron. In some embodiments, the polyoxometalates are magnetic, for example, ferromagnetic, diamagnetic (e.g., including copper or bismuth), paramagnetic, or superparamagnetic. Examples of polyoxometalates include [Co₄(H₂O)₂(PW₉O₃₄)₂]¹⁰⁻; [Co₄(H₂O)₂(P₂W₁₅O₅₆)₂]¹⁶⁻; Keggin POMs (such as [XM₁₂O₄₀]^{3-/4-}, where X can be P or Si, and M can be W, Mo, Fe, Mn, Cr, Ni, or Ru); Preyssler POMs (such as [M(H₂O)P₅W₃₀O₁₁₀]^{14/12-}, where M can be Na or Eu); Lindqvist POMs (such as [M₆O₁₉]²⁻); and Anderson POMs (such as [XM₆O₂₄]^{m-}). Other examples of polyoxometalates are described, for example, in Casan-Pastor et al., Frontiers in Bioscience 9, 1759-1770, May 1, 2004; Clemente-Leon et al., Adv. Mater. 2001, 13, No. 8, April 18, 574-577; Liu et al., Journal of Cluster Science, Vol. 14, No. 3, September 2003, 405-419; Hu Changwen et al., "Polyoxometalate-based Organic-inorganic Hybrid Materials" Chemical Journal on Internet, volume 3, number 6, page 22 (June 1, 2001); Kurth et al., Chem. Mater., 2000, 12, 2829-2831; and the entire issue of Chem. Rev. 1998, 98, 1. One or more layers 27 can include one type of molecular magnetic cluster or different types of clusters.

As indicated above, the polyoxometalates are nanosized. In some embodiments, the polyoxometalates have at least one dimension from about 50 nm (five Angstroms) to about 500 nm (fifty Angstroms).

For example, the polyoxometalates can have a dimension greater than or equal to about 50 nm (5 Å), about 100 nm (10 Å), about 150 nm (15 A), about 200 nm (20 A), about 250 nm (25 A), about 300 nm (30 Å), about 350 nm (35 Å), about 400 nm (40 Å), about 450 nm (45 Å); and/or less than or equal to about 500 nm (50 A), about 450 nm (45 A), about 400 nm (40 A), about 350 nm (35 A), about 300 nm (30 A), about 250 nm (25 A), about 200 nm (20 Å), about 150 nm (15 Å), about 100 nm (10 Å).

As shown in Fig. 2, layers 27 containing magnetic clusters 28 are separated by charged layers 30. The material of charged layers 30 also keep magnetic clusters 28 or groups of clusters spaced from each other. In some embodiments, charged layers 30 include a polyelectrolyte, for example, a positively charged polyelectrolyte to maintain charge balance with the negatively charged polyoxometalates. Polyelectrolytes are polymers having charged (e.g., ionically dissociable) groups. The number of these groups in the polyelectrolytes can be so large that the polymers are soluble in polar solvents (including water) when in ionically dissociated form (also called polyions). Depending on the type of dissociable groups, polyelectrolytes can be classified as polyacids and polybases. When dissociated, polyacids form polyanions, with protons being split off. Polyacids include inorganic, organic and biopolymers. Examples of polyacids are polyphosphoric acids, polyvinylsulfuric acids, polyvinylsulfonic acids, polyvinylphosphonic acids and polyacrylic acids. Examples of the corresponding salts, which are called polysalts, are polyphosphates, polyvinylsulfates, polyvinylsulfonates, polyvinylphosphonates and polyacrylates. Polybases contain groups that are capable of accepting protons, e.g., by reaction with acids, with a salt being formed. Examples of polybases having dissociable groups within their backbone and/or side groups are polyallylamine, polyethylimine, polyvinylamine and polyvinylpyridine. By accepting protons, polybases form polycations. Some polyelectrolytes have both anionic and cationic groups, but nonetheless have a net positive or negative charge.

The polyelectrolytes can include those based on biopolymers. Examples include alginic acid, gummi arabicurn, nucleic acids, pectins and proteins, chemically modified biopolymers such as carboxymethyl cellulose and lignin sulfonates, and synthetic polymers such as polymethacrylic acid, polyvinylsulfonic acid, polyvinylphosphonic acid and polyethylenimine. Linear or branched polyelectrolytes can be used. Using branched polyelectrolytes can lead to less compact polyelectrolyte multilayers having a higher degree of wall porosity. In some embodiments, polyelectrolyte molecules can be crosslinked within or/and between the individual layers, to enhance stability, e.g., by crosslinking amino groups with aldehydes. Furthermore, amphiphilic polyelectrolytes, e.g., amphiphilic block or random copolymers having partial polyelectrolyte character, can be used in some embodiments to affect permeability towards polar small molecules.

Other examples of polyelectrolytes include low-molecular weight polyelectrolytes (e.g., polyelectrolytes having molecular weights of a few hundred Daltons up to macromolecular polyelectrolytes (e.g., polyelectrolytes of synthetic or biological origin, which commonly have molecular weights of several million Daltons).

Still other examples of polyelectrolyte cations (polycations) include protamine sulfate polycations, poly(allylamine) polycations (e.g., poly(allylamine hydrochloride) (PAH)), polydiallyldimethylammonium polycations, polyethyleneimine polycations, chitosan polycations, gelatin polycations, spermidine polycations and albumin polycations. Examples of polyelectrolyte anions (polyanions) include poly(styrenesulfonate) polyanions (e.g., poly(sodium styrene sulfonate) (PSS)), polyacrylic acid polyanions, sodium alginate polyanions, eudragit polyanions, gelatin polyanions, hyaluronic acid polyanions, carrageenan polyanions, chondroitin sulfate polyanions, and carboxymethylcellulose polyanions.

In some embodiments, to increase the concentration of metallic elements in multi-layered structure 26, one or more charged layers 30 can include metallic elements. For example, multi-layered structure 26 can include a plurality of layers 27 containing anionic magnetic clusters having metallic element X, and a plurality of layers containing cationic species also having metallic element X (such as Fe(phen)₃²⁺ or Os(bpy)₃²⁺). In other embodiments, the cationic species can have different metallic elements than those of the anionic clusters. Examples of incorporating metal-containing species into a multi-layer structure are described in, for example, Liu et al., Journal of Cluster Science, Vol. 14, No. 3, September 2003, 405-419; and Moriguchi et al., Chem. Mater. 10, 2205 (1998).

In some embodiments, biodisintegrable polyelectrolytes can be used. For example, by using polyelectrolytes that are biodisintegrable near the outer surface of stent 20, a therapeutic agent can be released into the subject at a rate that is dependent upon the rate of disintegration of the polyelectrolyte layers. Biodisintegrable polyelectrolytes can also be used in embodiments in which the medical device is biodisintegrable. For example, stent 20 can include (e.g., be formed of) a biodisintegrable metal or a biodisintegrable polymer, as described in Bolz, U.S. 6,287,332; Heublein, US 2002/0004060 Al; U.S. 5,587,507; and U.S. 6,475,477. As used herein, a "biodisintegrable material" is a material that undergoes dissolution, degradation, absorption, erosion, corrosion, resorption and/or other disintegration processes over the period that the device is designed to reside in a patient. In other embodiments, biostable polyelectrolytes are utilized. As used herein, a "biostable material" is a material that does not undergo substantial dissolution, degradation, corrosion, resorption and/or other disintegration processes over the period that the device is designed to reside in a patient. Examples of biodisintegrable and biostable polyelectrolytes include polyglycolic acid (PGA), polylactic acid (PLA), polyamides, poly-2-hydroxy-butyrate (PHB), polycaprolactone (PCL), poly(lactic-co-glycolic)acid (PLGA), protamine sulfate, polyallylamine, polydiallyldimethylammonium species, polyethyleneimine, chitosan, eudragit, gelatin, spermidine, albumin, polyacrylic acid, sodium alginate, polystyrene sulfonate, hyaluronic acid, carrageenan, chondroitin sulfate, and carboxymethylcellulose. One or more charged layers 30 can include one type of polyelectrolyte or different types of polyelectrolytes.

Charged layers 30 containing the polyelectrolytes can be assembled with layers 27 containing magnetic clusters 28 using a layer-by-layer technique in which the layers electrostatically self-assemble. In the layer-by-layer technique, a first layer having a first surface charge is deposited on an underlying substrate, followed by a second layer having a second surface charge that is opposite in sign to the surface charge of the first layer. Thus, the charge on the outer layer is reversed upon deposition of each sequential layer. Additional first and second layers can then be alternatingly deposited on the substrate to build multi-layered structure 26 to a predetermined or targeted thickness. The layer-by-layer technique allows structure 26 to be formed on tubular structure 21 directly and/or, for example, on a flexible sleeve (e.g., a polymer sleeve) carried by the tubular structure. As a result, structure 26 is capable of enhancing the MRI compatibility of stent 20, while allowing the stent to remain flexible and adaptable to the vessel in which is stent is implanted. Examples of incorporating polyoxometalates in a multi-layered structured using a layer-by-layer technique is described, for example, in Caruso et al., Langmuir 1998,14, 3462-3465.

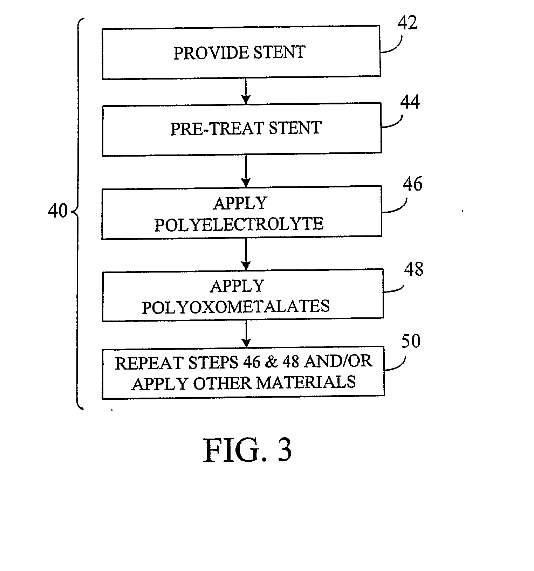

Referring to Fig. 3, an embodiment of a method 40 of making stent 20 using a layer-by-layer technique is shown. Method 40 includes providing a starting stent (step 42) and pretreating the starting stent for layer-by-layer deposition (step 44). Next, a charged layer 30 containing a polyelectrolyte can be applied on the starting stent (step 46). A layer 27 containing magnetic clusters 28 can then be applied to the previously applied charged layer 30 (step 48). Steps 46 and 48 can then repeated to build a multi-layered structure 26 of a desired thickness to form stent 20. In some embodiments, as described below, multi-layered structure 26 can further include one or more layers including a therapeutic agent, one or more layers including a radiopaque material, and/or one or more layers capable of enhancing the mechanical properties of structure 26. These additional layers can be applied between layers 27 and/or layers 30, in any combination. Layer-by-layer self-assembly is described, for example, in Liu et al., Journal of Cluster Science, Vol. 14, No. 3, September 2003, 405-419; and Caruso et al., Langmuir 1998, 14, 3462-3465..

The starting stent can be manufactured, or the starting stent can be obtained commercially. Methods of making stents are described, for example, in U.S. Patent No. 5,780,807 and U.S. Application Publication US-2004-0000046-A1. Stents are also available, for example, from Boston Scientific Corporation.

The provided stent can be formed of any biocompatible material, e.g., a metal or an alloy. The biocompatible material can be suitable for use in a self-expandable stent, a balloon-expandable stent, or both. For self-expandable stents, the stent can be formed of a continuous solid mass of a relatively elastic biocompatible material, such as a superelastic or pseudo-elastic metal alloy, for example, a Nitinol (e.g., 55% nickel, 45% titanium). Examples of materials that can be used for a balloon-expandable stent include noble metals, radiopaque materials, stainless steel, and alloys including stainless steel and one or more radiopaque materials. Specific examples of biocompatible materials are described U.S. Application Publications US-2004-0230290-A1, US-2003-0018380-A1, US-2002-0144757-A1, and US-2003-0077200-A1.

Next, the provided stent can be pretreated (step 44). For example, the stent can be cleaned to remove surface contaminants, such as oil, that can affect the homogeneity to which multi-layered structure 26 can be formed. The stent can be cleaned, for example, in a mixture such as acetone, H₂O₂/HCl, HCl/HNO₃, H₂SO₄/K₂Cr₂O₇, H₂O₂/NH₃, and/or NaOH/NaOCl. The stent can also be pretreated with a solution including 10⁻² M SDS/0.12 N HCl for 15 minutes at 100°C.

Next, while certain stent materials may be inherently charged and thus lend themselves to layer-by-layer assembly, to the extent that the stent does not have an inherent net surface charge, a surface charge may be provided. For example, where the stent to be coated is conductive, a surface charge can be provided by applying an electrical potential to the stent. Once a first polyelectrolyte layer is applied in this fashion, a second polyelectrolyte layer having a second surface charge that is opposite in sign to the surface charge of the first polyelectrolyte layer, or a layer containing magnetic clusters, can be applied, and so forth.

As another example, the stent can be provided with a positive charge by covalently attaching functional groups having positive charge (e.g., amine, imine or other basic groups) or functional groups having a negative charge (e.g., carboxylic, phosphonic, phosphoric, sulfuric, sulfonic, or other acid groups).

As another example, a surface charge can be provided by exposing the stent to a charged amphiphilic substance. Amphiphilic substances can include any substance having hydrophilic and hydrophobic groups. In embodiments, the amphiphilic substance includes at least one electrically charged group to provide the stent surface with a net electrical charge. Therefore, the amphiphilic substances that are used herein can also be referred to as ionic amphiphilic substances.

Amphiphilic polyelectrolytes can be used as ionic amphiphilic substances in some embodiments. For example, a polyelectrolyte comprising charged groups (which are hydrophilic) as well as hydrophobic groups, such as polyethylenimine (PEI) or poly(styrene sulfonate) (PSS), can be employed. Cationic and anionic surfactants are also used as amphiphilic substances in some embodiments. Cationic surfactants include quaternary ammonium salts (R₄N⁺X⁻), where R is an organic radical and where X- is a counter-anion, e. g. a halogenide, for example, didodecyldimethylammonium bromide (DDDAB), alkyltrimethylammonium bromides such as hexadecyltrimethylammonium bromide (HDTAB), dodecyltrimethylammonium bromide (DTMAB), myristyltrimethylammonium bromide (MTMAB), or palmityl trimethylammonium bromide, or N-alkylpyridinium salts, or tertiary amines (R₃NH⁺X⁻), for example, cholesteryl-3β-N-(dimethyl-aminoethyl)-carbamate or mixtures thereof. Anionic surfactants include alkyl or olefin sulfate (R--OSO₃M), for example, a dodecyl sulfate such as sodium dodecyl sulfate (SDS), a lauryl sulfate such as sodium lauryl sulfate (SLS), or an alkyl or olefin sulfonate (R--SO₃M), for example, sodium-n-dodecylbenzene sulfonate, or fatty acids (R--COOM), for example, dodecanoic acid sodium salt, or phosphoric acids or cholic acids or fluoro-organics, for example, lithium-3-[2-(perfluoroalkyl)ethylthio]propionate or mixtures thereof, where R is an organic radical and M is a counter-cation.

Thus, a surface charge can be provided on the stent by adsorbing cations (e.g., protamine sulfate, polyallylamine, polydiallyldimethylammonium species, polyethyleneimine, chitosan, gelatin, spermidine, and/or albumin) or by adsorbing anions (e.g., polyacrylic acid, sodium alginate, polystyrene sulfonate, eudragit, gelatin (an amphiphilic polymer that fits in both categories depending how it is being prepared), hyaluronic acid, carrageenan, chondroitin sulfate, and/or carboxymethylcellulose) to the surface of the stent as a first charged layer. As an example, poly(ethylene imine) (PEI, Aldrich, MW ∼25 kD) can be dissolved in water in a concentration of about 0.5 g/L to apply a first coating. In some embodiments, more than one surface charge layer can be applied to provide complete coverage of the stent. Application of surface charge layers is described in, e.g., "Multilayer on Solid Planar Substrates" Multi-layer Thin Films, Sequential Assembly of Nanocomposite Materials, Wiley-VCH ISBN 3-527-30440-1, Chapter 14; and "Surface-chemistry Technology for Microfluidics" Hau, Winky L. W. et al., J. Micromech. Microeng. 13 (2003) 272-278.

The species for establishing a surface charge can be applied to the stent by a variety of techniques. Examples of techniques include spraying techniques, dipping techniques, roll and brush coating techniques, techniques involving coating via mechanical suspension such as air suspension, ink jet techniques, spin coating techniques, web coating techniques and combinations of these processes. Dipping and spraying techniques (without masking) can be employed, for example, to apply the species to an entire stent. Roll coating, brush coating and ink jet printing can be employed, for example, to apply the species only to selected portions of the stent (e.g., in the form of a pattern).

Once a preselected charge is provided on the stent, the stent can be coated with a layer of an oppositely charged material. After each application of a layer, the stent can be washed to remove excess material. Multi-layer structure 26 can be formed by repeated treatment with alternating, oppositely charged materials, e.g., by alternating treatment of a positive polyelectrolyte with treatment with a negative polyoxometalate to build layers 27 and 30 (steps 46 and 48). Layers 27 and 30 self-assemble by electrostatic layer-by-layer deposition, thus forming multi-layered structure 26 over the stent.

As indicated above, in some embodiments, multi-layered structure 26 can further include one or more layers including a therapeutic agent, one or more layers including a radiopaque material, and/or one or more layers capable of enhancing the mechanical properties of structure 26.

As an example, one or more therapeutic agents can be disposed on or within multi-layered structure 26 giving the medical device, for example, a drug releasing function upon implantation. The therapeutic agent can be charged, for example, because it is itself a charged molecule or because it is intimately associated with a charged molecule. Examples of charged therapeutic agents include small molecule and polymeric therapeutic agents containing ionically dissociable groups. In embodiments in which the therapeutic agent does not possess one or more charged groups, it can nevertheless be provided with a charge, for example, through non-covalent association with a charged species. Examples of non-covalent associations include hydrogen bonding, and hydrophilic/lipophilic interactions. For instance, the therapeutic agent can be associated with an ionic amphiphilic substance.

In certain embodiments in which a charged therapeutic agent is used, one or more layers of the charged therapeutic agent are deposited during the course of assembling multi-layer structure 26. For example, the therapeutic agent can be a polyelectrolyte (e.g., where the therapeutic agent is a polypeptide or a polynucleotide) and it is used to create one or more polyelectrolyte layers within multi-layer structure 26. In other embodiments, the charged therapeutic agent is not a polyelectrolyte (e.g., it may be a charged small molecule drug), but one or more layers of the charged therapeutic agent can be substituted for one or more layers of the same charge (i.e., positive or negative) during the layer-by-layer assembly process.

In still other embodiments, the therapeutic agent can provided within charged nanocapsules, which are formed, for example, using layer-by-layer techniques such as those described herein and in commonly assigned US-2005-0129727-A1, entitled "Localized Drug Delivery Using Drug-Loaded Nanocapsules". In these embodiments, one or more layers of the charged nanocapsules can be deposited during the course of the layer-by-layer assembly process.

In still other embodiments, multi-layer structure 26 is loaded with a therapeutic agent subsequent to its formation. For example, the porosity, and thus the permeability, of structure 26 can be modified by modifying the pH exposed to the structure, as described, for example, in Antipov, A. A. et al., "Polyelectrolyte multilayer capsule permeability control, " Colloids and Surfaces A: Physicochemical and Engineering Aspects, 198-200 (2002) pp. 535-541.

Examples of therapeutic agents and methods of incorporated the agents are described in US-2010-0179645-A1 and U. S. Patent No. 5,733,925.

Some examples of therapeutic agents include non-genetic therapeutic agents, genetic therapeutic agents, vectors for delivery of genetic therapeutic agents, cells, and therapeutic agents identified as candidates for vascular treatment regimens, for example, as agents targeting restenosis,

To enhance the radiopacity of stent 20, a radiopaque material, such as gold nanoparticles, can be incorporated into multi-layered structure 26. For example, gold nanoparticles can be made positively charged by applying a outer layer of lysine to the nanoparticles, e.g., as described in "DNA Mediated Electrostatic Assembly of Gold Nanoparticles into Linear Arrays by a Simple Dropcoating Procedure" Murali Sastrya and Ashavani Kumar, Applied Physics Letters, Vol. 78, No. 19, 7 May 2001. Other radiopaque materials include, for example, tantalum, platinum, palladium, tungsten, iridium, and their alloys.

In some embodiments, multi-layered structure 26 includes nanoparticles that can enhance the mechanical properties, e.g., strength, of the structure. The nanoparticles can have at least one dimension (e.g., the thickness for a nanoplate, the diameter for a nanosphere, a nanocylinder and a nanotube) that is less than 1000 nm, e.g., less than 100 nm. Nanoplates can have at least one dimension that is less than 1000 nm; nanofibers can have at least two orthogonal dimensions (e.g., the diameter for a cylindrical nanofiber) that are less than 1000 nm; and other nanoparticles can have three orthogonal dimensions that are less than 1000 nm (e.g., the diameter for nanospheres).

Examples of nanoparticles include carbon, ceramic and metallic nanoparticles including nanoplates, nanotubes, and nanospheres, and other nanoparticles. Specific examples of nanoplates include synthetic or natural phyllosilicates including clays and micas (which may optionally be intercalated and/or exfoliated) such as montmorillonite, hectorite, hydrotalcite, vermiculite and laponite. Specific examples of nanotubes and nanofibers include single-wall and multi-wall carbon nanotubes, such as fullerene nanotubes, vapor grown carbon fibers, alumina nanofibers, titanium oxide nanofibers, tungsten oxide nanofibers, tantalum oxide nanofibers, zirconium oxide nanofibers, and silicate nanofibers such as aluminum silicate nanofibers. Other examples of nanoparticles (e. g., nanoparticles having three orthogonal dimensions that are less than 1000 nm) include fullerenes (e.g., bucky balls), silica nanoparticles, aluminum oxide nanoparticles, titanium oxide nanoparticles, tungsten oxide nanoparticles, tantalum oxide nanoparticles, zirconium oxide nanoparticles, dendrimers, and monomeric silicates such as polyhedral oligomeric silsequioxanes (POSS), including various functionalized POSS and polymerized POSS. The carbon nanotubes and carbon nanofibers can have a diameter ranging from 0.5 nm to 200 nm. Still other examples of nanoparticles include compositions of the Mo-SI family, such as Mo₆S₃I₆ and Mo₃S₆I, which are described in Vrbanić et al., Nanotechnology 15 (2004) 635-638; Rem 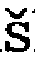 kar et al., *Science* 292 (2001) 479; and Mihailović et al., Phys. Rev. Lett. 90 (2003) 146401-1. Mo₃S₆I, for example when doped, can exhibit a large paramagnetic susceptibility, which can further enhance the magnetic shielding capability of the multi-layered structure.

Various techniques can be used to provide charges on nanoparticles that are not inherently charged. For example, a surface charge can be provided by adsorbing or otherwise attaching species on the nanoparticles that have a net positive or negative charge, for example, charged amphiphilic substance such as amphiphilic polyelectrolytes and cationic and anionic surfactants (see above). Where the nanoparticles are sufficiently stable, surface charges can sometimes be established by exposure to highly acidic conditions. For example, carbon nanoparticles, such as carbon nanotubes, can be partially oxidized by refluxing in strong acid to form carboxylic acid groups (which ionize to become negatively charged carboxyl groups) on the nanoparticles. Establishing a surface charge on nanoparticles can also provide a relatively stable and uniform suspension of the nanoparticles, due at least in part to electrostatic stabilization effects. Layer-by-layer assembly to form alternating layers of SWNT and polymeric material have also been described, e.g., in Arif A. Mamedov et al., "Molecular Design of Strong Singlewall Carbon Nanotube/Polyelectrolyte Multilayer Composites" Nature Material, Vol. 1, No. 3, 2002, pages 191-194. The nanoparticles can be positively charged or negatively charged, and both types of nanoparticles can be used in a medical device. A medical device can include one composition of nanoparticles or different compositions of nanoparticles.

The layer-by-layer assembly can be conducted by exposing a selected charged substrate (e.g., stent) to solutions or suspensions that contain species of alternating net charge, including solutions or suspensions that contain charged magnetic clusters, charged polyelectrolytes, and, optionally, charged therapeutic agents and/or nanoparticles. The concentration of the charged species within these solutions and suspensions, which can be dependent on the types of species being deposited, can range, for example, from about 0.01 mg/ml to about 30 mg/ml. The pH of these suspensions and solutions can be such that the magnetic clusters, polyelectrolytes, and optional therapeutic agents and/or nanoparticles maintain their charge. Buffer systems can be used to maintain charge.

The solutions and suspensions containing the charged species (e.g., solutions/suspensions of magnetic clusters, polyelectrolytes, or other optional charged species such as charged therapeutic agents and/or charged nanoparticles) can be applied to the charged substrate surface using a variety of techniques. Examples of techniques include spraying techniques, dipping techniques, roll and brush coating techniques, techniques involving coating via mechanical suspension such as air suspension, ink jet techniques, spin coating techniques, web coating techniques and combinations of these processes. Layers can be applied over an underlying substrate by immersing the entire substrate (e.g., stent) into a solution or suspension containing the charged species, or by immersing half of the substrate into the solution or suspension, flipping the same, and immersing the other half of the substrate into the solution or suspension to complete the coating. In some embodiments, the substrate is rinsed after application of each charged species layer, for example, using a washing solution with a pH that maintains the charge of the outer layer.

In some embodiments, to prevent the polyelectrolyte layers from dissolving in the body, one or more of the top polyelectrolyte layers can be cross-linked. For example, multiple layers of polyallylamine hydrochloride (PAH) and polyacrylic acid (PAA) can be deposited on a plurality of layers containing POMs alternating with a plurality of cationic layers. The entire multi-layered structure can then be heated at 130°C for about an hour under a nitrogen atmosphere to crosslink the ammonium groups of the PAH and the carboxylic groups of the PAA to form amide bonds. A nylon-like top film that is not permeable to liquids can be created.

Using the techniques described herein, multiple layers of alternating charge can be applied over the underlying substrate, including the application of one or more charged layer 27 and the application of one or more charged polyelectrolyte layer 30. The number of each layer and/or the total thickness of multi-layered structure 26 can be determined empirically and can be a function of, for example, the compositions of the layers and the type of medical device. For example, for a given medical device, the number of layers, their sequences and compositions, and/or the total thickness of multi-layered structure 26 can be varied and the effectiveness of the multi-layered structure can be tested. After an effective combination is determined, the same combination can be repeated. In some embodiments, between 10 and 300 layers are applied over the substrate. The total thickness of multi-layered structure 26 can be a function of the materials (e.g., POMs and/or polyelectrolytes) used, and can range, for example, from 5 nanometers to 1500 nanometers.

In use, stent 20 can be delivered and expanded using, for example, a balloon catheter system or other stent delivery systems. Catheter systems are described in, for example, Wang U.S. 5,195,969, and Hamlin U.S. 5,270,086. Stents and stent delivery are also exemplified by the Radius® or Symbiot® systems, available from Boston Scientific Scimed, Maple Grove, MN.

Stent 20 can be of any desired shape and size (e.g., coronary stents, aortic stents, peripheral vascular stents, gastrointestinal stents, urology stents, and neurology stents). Depending on the application, stent 20 can have a diameter of between, for example, 1 mm to 46 mm. In certain embodiments, a coronary stent can have an expanded diameter of from about 2 mm to about 6 mm. In some embodiments, a peripheral stent can have an expanded diameter of from about 4 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. Stent 20 can be balloon-expandable, self-expandable, or a combination of both (e.g., U.S. Patent No. 5,366,504).

In some embodiments, polyelectrolyte layers on both sides of a polyoxometalate layer can be bonded together. For example, multi-layered structure 26 can include a layer including polyallylamine, a layer including polyoxometalates, and a layer including polyacrylic acid. The polyallylamine and the polyacrylic acid can be crosslinked to form a polyamide that enhances the integrity of multi-layered structure 26.

As another example, multiple polyelectrolyte layers can be formed between layers including polyoxometalates.

Others methods of incorporating molecular magnetic clusters such as polyoxometalates can also be used. For example, the polyoxometalates can be included in a mixture having one or more polymerizable materials, and the mixture can be polymerized to encapsulate the polyoxometalates in a polymer. More specifically, the polyoxometalates can be mixed with a monomer (such as pyrrole, aniline, and/or thiophene), and the mixture can be electropolymerized using a medical device (such as a stent) as an electrode. Upon polymerization, the polyoxometalates can be encapsulated by a conducting polymer on the device. Polymerization of a polymer and a polyoxometalate is described, for example, in Cheng et al., Synthetic Materials 129 (2002) 53-59; and A.M. White and R.C.T. Slade, Synthetic Metals, 8 August 2003, vol. 139, issue 1, pp. 123-131(9). For example, films can be prepared by electrochemical oxidation at a constant anodic potential of 1.2 V (vs. a Ag/AgCl electrode) by passing 100 mC through a one-compartment cell including a monomeric solution and three electrodes. The solution can contain 0.1 M pyrrole and 0.001 M TBA₃[PW₁₂₋ₓMoₓO₄₀] (x=0, 3, 6, 12) in acetonitrile.

Alternatively or additionally, in some embodiments, the polymer can be used to encapsulate one or more nanoparticles, such as magnetic nanoparticles (e.g., CoFe₂O₄) and those described herein. Such polymer/magnetic nanoparticles systems are described in Sing et al., Electrochimica Acta 49 (2004) 4605-4612. The polymer/nanoparticles composite can enhance the mechanical properties of the structure and/or enhance the magnetic shielding effect, for example.

In embodiments, the polymer (such as polypyrrole (PPY) can be doped with inorganic anions and/or polymeric anions. For example, to prepare a PPY⁺ClO₄⁻ film, an acetonitrile (AN) solution containing 0.2 mol/dm³ pyrrole and 0.2 mol/dm³ LiClO₄ can be used. To prepare a polypyrrole film with polymeric anions, such as poly(vinylsulfonate) (PVC) and poly(styrenesufonate) (PSS), aqueous solutions containing 0.1 mol dm⁻³ KPVC or 0.01 mol dm⁻³ of NaPSS can be used. The solutions can be purged with N₂. PPY⁺ClO₄⁻ and PPY/PSS films can be made by using electrooxidative polymerization at multiple potentials, in the range from 0.4 V to 1.2 V vs. Ag/AgNO₃ or a saturated calomel electrode. Examples of electropolymerization of conducting polymers are described, for example, in Atanasoska et al., Chem. of Mater., 1992, 4, 988. Conducting polymers may have a beneficial effect on cell interaction and thus on endothelialization of the stent after implantation.

In some embodiments, multi-layered structure 26 can be formed on a substrate, removed from the substrate, and subsequently applied (e.g., with an adhesive) to a medical device. The substrate can be removed by destroying it, for example, by melting, sublimation, combustion, and/or dissolution, to free multi-layered structure 26. For example, a removable substrate made of dental waxes (such as those available from MDL Dental Products, Inc., Seattle, WA, USA) or polyvinyl alcohol (PVOH) can be used. These materials can melt at moderately elevated temperatures (e.g., 60°C) and dissolve in hot water, respectively. Other methods of using a removable substrate are described in Sukhorukov et al., "Comparative Analysis of Hollow and Filled Polyelectrolyte Microcapsules Templated on Melamine Formaldehyde and Carbonate Cores, Macromol." Chem. Phys., 205, 2004, pp. 530-535; and U.S.S.N. 10/849742.

As another example, one or more reinforcement aids can be provided adjacent to or within multi-layered structure 26 to enhance its mechanical properties. For example, one or more reinforcement aids can be applied to a substrate, followed by a series of polyelectrolyte and polyoxometalates layers. As another example, a first series of polyelectrolyte layers or a first series of both polyelectrolyte and polyoxometalates layers can be provided, followed by the application of one or more reinforcement aids, followed by a second series of polyelectrolyte layers or a second series of both polyelectrolyte and polyoxometalates layers. Examples of reinforcement aids include fibrous reinforcement members such as metal fiber meshes, metal fiber braids, metal fiber windings, intermingled fibers (e.g., metal fiber, carbon fibers, high density polyethylene fibers, liquid polymer crystals) and so forth. The reinforcement aids can be provided with a surface charge to enhance incorporation of the reinforcement aids onto or into multi-layered structure 26. For example, layer-by-layer techniques can be used to encapsulate the reinforcement aids, thereby providing them with a charged outer layer and enhancing interaction of the reinforcement aids with an adjacent layer (e.g., a polyelectrolyte or polyoxometalate layer) of opposite charge. The loading of the reinforcement aids can be such that they do form a conductive loop or solenoid.

Additionally to the polyoxometalates, other ionic molecular species containing metals can be used. Examples include molybdenum selenocyanide anions such as [Mo₆Se₈(CN)₆]⁷⁻ and [Mo₆Se₈(CN)₆]⁶⁻, for example, as described in Mironov et al., Chem. Eur. J. 2000, 6, No. 8, 1361-1365*.*

Stent 20 can also be a part of a stent-graft or a covered stent. In other embodiments, stent 20 can include and/or be attached to a biocompatible, non-porous or semi-porous polymer matrix made of polytetrafluoroethylene (PTFE), expanded PTFE, polyethylene, urethane, or polypropylene.

Other embodiments are within the claims.

## Claims

1. An endoprosthesis comprising a plurality of polyoxometalates.

2. The endoprosthesis of claim 1, wherein the device comprises a layer comprising the polyoxometalates.

3. The endoprosthesis of claim 2, comprising a plurality of layers comprising the polyoxometalates, the layers being spaced from each other.

4. The endoprosthesis of claim 3, further comprising a first layer comprising a polyelectrolyte, the first layer being between the layers comprising the polyoxometalates.

5. The endoprosthesis of claim 1, comprising a layer comprising the polyoxometalates electrostatically bonded to a layer comprising one or more charges.

6. The endoprosthesis of claim 1, comprising a first layer comprising a first polymer, a second layer comprising a second polymer, and a third layer comprising the polyoxometalates between the first layer and the second layer, wherein the first polymer and the second polymer are covalently bonded to each other.

7. The endoprosthesis of claim 1, wherein the polyoxometalates are ferromagnetic.

8. The endoprosthesis of claim 1, wherein the polyoxometalates are superparamagnetic, paramagnetic, or diamagnetic.

9. The endoprosthesis of claim 1, wherein the polyoxometalates comprise an element selected from the group consisting of cobalt, iron, manganese, chromium, nickel, ruthenium, copper, and bismuth.

10. The endoprosthesis of claim 1, wherein the polyoxometalates have at least one dimension of about 0.5 nm to about 50 nm.

11. The endoprosthesis of claim 1, wherein the polyoxometalates are surrounded by a polymer.

12. The endoprosthesis of claim 10, wherein the polymer is selected from the group consisting of polypyrrole, polythiophene, and polyaniline.

13. A method of making an endoprosthesis, the method comprising:
forming on the endoprosthesis a first layer comprising one or more positive charges; and
forming on the first layer a second layer comprising polyoxometalates.

14. The method of claim 13, further comprising forming a plurality of first layers and a plurality of second layers.

15. The method of claim 13, wherein the first layer comprises a polyelectrolyte.

16. The method of claim 13, wherein the polyoxometalates are ferromagnetic, diamagnetic, paramagnetic, or superparamagnetic.

17. The method of claim 13, wherein the polyoxometalates comprise an element selected from the group consisting of cobalt, iron, manganese, chromium, nickel, ruthenium, copper, and bismuth.

## Patentansprüche

1. Eine Endoprothese umfassend eine Vielzahl von Polyoxometalaten.

2. Die Endoprothese nach Anspruch 1, wobei die Vorrichtung eine Schicht umfasst, die Polyoxometalate umfasst.

3. Die Endoprothese nach Anspruch 2, umfassend eine Vielzahl von Schichten, die Polyoxometalate umfassen, wobei die Schichten voneinander beabstandet sind.

4. Die Endoprothese nach Anspruch 3, weiter umfassend eine erste Schicht, die ein Polyelektrolyt umfasst, wobei die erste Schicht zwischen den Schichten ist, die die Polyoxometalate umfassen.

5. Die Endoprothese nach Anspruch 1, umfassend eine Schicht, die Polyoxometalate umfasst, das elektrostatisch gebunden ist an eine Schicht, die eine oder mehr Ladungen umfasst.

6. Die Endoprothese nach Anspruch 1, umfassend eine erste Schicht, die ein erstes Polymer umfasst, eine zweite Schicht, die ein zweites Polymer umfasst und eine dritte Schicht, die Polyoxometalate umfasst, zwischen der ersten Schicht und der zweiten Schicht, wobei das erste Polymer und das zweite Polymer kovalent aneinander gebunden sind.

7. Die Endoprothese nach Anspruch 1, wobei die Polyoxometalate ferromagnetisch sind.

8. Die Endoprothese nach Anspruch 1, wobei die Polyoxometalate superparamagnetisch, paramagnetisch oder diamagnetisch sind.

9. Die Endoprothese nach Anspruch 1, wobei die Polyoxometalate ein Element ausgewählt aus der Gruppe bestehend aus Cobalt, Eisen, Mangan, Chrom, Nickel, Ruthenium, Kupfer und Bismut umfassen.

10. Die Endoprothese nach Anspruch 1, wobei die Polyoxometalate eine Größe von ungefähr 0.5 nm bis ungefähr 50 nm haben

11. Die Endoprothese nach Anspruch 1, wobei die Polyoxometalate von einem Polymer umgeben sind.

12. Die Endoprothese nach Anspruch 10, wobei das Polymer ausgewählt ist aus der Gruppe bestehend aus Polypyrrolen, Polythiophenen und Polyanilinen.

13. Ein Verfahren zum Herstellen einer Endoprothese, wobei das Verfahren umfasst:
Formen einer ersten Schicht umfassend einer oder mehrerer positiver Ladungen auf einer Endoprothese; und
Formen einer zweiten Schicht umfassend Polyoxometalate auf der ersten Schicht.

14. Das Verfahren nach Anspruch 13, weiter umfassend Formen einer Vielzahl von ersten Schichten und einer Vielzahl von zweiten Schichten.

15. Das Verfahren nach Anspruch 13, wobei die erste Schicht ein Polyelektrolyt umfasst.

16. Das Verfahren nach Anspruch 13, wobei die Polyoxometalate ferromagnetisch, diamagnetisch, paramagnetisch oder superparamagnetisch sind.

17. Das Verfahren nach Anspruch 13, wobei die Polyoxometalate ein Element ausgewählt aus der Gruppe bestehend aus Cobalt, Eisen, Mangan, Chrom, Nickel, Ruthenium, Kupfer und Bismut umfassen.

## Revendications

1. Endoprothèse comprenant une pluralité de polyoxométallates.

2. Endoprothèse selon la revendication 1, le dispositif comprenant une couche comprenant les polyoxométallates.

3. Endoprothèse selon la revendication 2, comprenant une pluralité de couches comprenant les polyoxométallates, les couches étant espacées les unes des autres.

4. Endoprothèse selon la revendication 3, comprenant en outre une première couche comprenant un polyélectrolyte, la première couche se situant entre les couches comprenant les polyoxométallates.

5. Endoprothèse selon la revendication 1, comprenant une couche comprenant les polyoxométallates liée électrostatiquement à une couche comprenant une ou plusieurs charges.

6. Endoprothèse selon la revendication 1, comprenant une première couche comprenant un premier polymère, une deuxième couche comprenant un deuxième polymère, et une troisième couche comprenant les polyoxométallates entre la première couche et la deuxième couche, le premier polymère et le deuxième polymère étant liés l'un à l'autre de façon covalente.

7. Endoprothèse selon la revendication 1, dans laquelle les polyoxométallates sont ferromagnétiques.

8. Endoprothèse selon la revendication 1, dans laquelle les polyoxométallates sont superparamagnétiques, paramagnétiques, ou diamagnétiques.

9. Endoprothèse selon la revendication 1, dans laquelle les polyoxométallates comprennent un élément choisi dans le groupe constitué par le cobalt, le fer, le manganèse, le chrome, le nickel, le ruthénium, le cuivre, et le bismuth.

10. Endoprothèse selon la revendication 1, dans laquelle les polyoxométallates ont au moins une dimension d'environ 0,5 nm environ 50 nm.

11. Endoprothèse selon la revendication 1, dans laquelle les polyoxométallates sont entourés d'un polymère.

12. Endoprothèse selon la revendication 10, dans laquelle le polymère est choisi dans le groupe constitué par le polypyrrole, le polythiophène, et la polyaniline.

13. Procédé de fabrication d'une endoprothèse, le procédé comprenant :
la formation sur l'endoprothèse d'une première couche comprenant une ou plusieurs charges positives ; et
la formation sur la première couche d'une deuxième couche comprenant des polyoxométallates.

14. Procédé selon la revendication 13, comprenant en outre la formation d'une pluralité de premières couches et d'une pluralité de deuxièmes couches.

15. Procédé selon la revendication 13, dans lequel la première couche comprend un polyélectrolyte.

16. Procédé selon la revendication 13, dans lequel les polyoxométallates sont ferromagnétiques, diamagnétiques, paramagnétiques, ou superparamagnétiques.

17. Procédé selon la revendication 13, dans lequel les polyoxométallates comprennent un élément choisi dans le groupe constitué par le cobalt, le fer, le manganèse, le chrome, le nickel, le ruthénium, le cuivre, et le bismuth.
